Europäisches Patentamt

European Patent Office

Office européen des brevets

(19)

(11) Numéro de publication : **0 158 758**
**B1**

(12) **FASCICULE DE BREVET EUROPÉEN**

(45) Date de publication du fascicule du brevet :
28.09.88

(21) Numéro de dépôt : 84402765.6

(22) Date de dépôt : 28.12.84

(51) Int. Cl.⁴ : **C 07 H 21/00**, C 12 Q 1/68,
G 01 N 33/53

(54) **Sonde contenant un acide nucléique modifié et reconnaissable par des anticorps spécifiques et utilisation de cette sonde et de ces anticorps spécifiques pour détecter et caractériser une séquence d' ADN homologue.**

(30) Priorité : 16.01.84 FR 8400607

(43) Date de publication de la demande :
23.10.85 Bulletin 85/43

(45) Mention de la délivrance du brevet :
28.09.88 Bulletin 88/39

(84) Etats contractants désignés :
AT BE CH DE GB IT LI LU NL SE

(56) Documents cités :
EP-A- 0 128 018
WO-A-83 /022 86
CHEMICAL ABSTRACTS, vol. 92, no. 13, 31 mars 1980, page 180, no. 105510u, Columbus, Ohio, US; E. SAGE et al.: "Discrimination by antibodies between local defects in DNA induced by 2-aminofluorene derivatives"

(73) Titulaire : **INSTITUT NATIONAL DE LA SANTE ET DE LA RECHERCHE MEDICALE (INSERM)**
**101, rue de Tolbiac**
**F-75654 Paris Cedex 13 (FR)**

**INSTITUT PASTEUR**
**25-28, rue du Docteur Roux**
**F-75724 Paris Cedex 15 (FR)**

(72) Inventeur : **Tchen, Paul**
**18, rue du Télégraphe**
**F-92000 Nanterre (FR)**
Inventeur : **Fuchs, Robert**
**20, rue de la Dordogne**
**F-67150 Gerstheim (FR)**
Inventeur : **Leng, Marc**
**50, rue de la Raffinerie**
**F-45590 Saint Cyr en Val (FR)**

(74) Mandataire : **Gutmann, Ernest et al**
**S.C. Ernest Gutmann - Yves Plasseraud 67, boulevard Haussmann**
**F-75008 Paris (FR)**

## Description

L'invention est relative à une sonde de haute sensibilité contenant un acide nucléique modifié, et susceptible d'être reconnue par des anticorps spécifiques ainsi qu'à l'utilisation de cette sonde pour la détection et la caractérisation d'une séquence d'acide nucléique homologue spécifique, notamment d'un ADN ou ARN, cellulaire ou viral, dans un échantillon susceptible de la contenir.

L'invention vise plus particulièrement une sonde modifiée chimiquement, de telle sorte qu'elle puisse, après hybridation avec la séquence d'acide nucléique homologue recherchée, être détectée par des anticorps spécifiques à l'égard de la sonde elle-même.

L'invention vise également un ensemble, nécessaire ou « kit » pour la détection et la caractérisation d'une séquence d'acide nucléique d'ADN ou d'ARN, cellulaire ou viral, lequel nécessaire comprend, d'une part, une sonde contenant un acide nucléique modifié, homologue de la séquence de l'acide nucléique modifié, homologue de l'acide nucléique spécifique recherché, et, d'autre part, les anticorps spécifiques à l'égard de la sonde elle-même, aptes à détecter la sonde après hybridation de celle-ci avec la séquence d'acide nucléique spécifique recherchée.

L'invention vise enfin un procédé de détection et de caractérisation d'une séquence spécifique ou déterminée d'acide nucléique d'ADN ou d'ARN mettant en œuvre la susdite sonde.

On a déjà décrit des sondes marquées permettant de détecter une séquence d'acide nucléique spécifique, telle qu'un gène ou un fragment de gène au sein d'une composition susceptible de la contenir, de telles sondes contenant alors une séquence d'ADN consistant en un acide nucléique complémentaire, susceptible de s'hybrider avec la séquence d'acide nucléique ou le gène recherché. Le marqueur est avantageusement formé par un groupe de modification porté par la sonde, ce groupe de modification (ou l'ADN ainsi modifié) pouvant alors être reconnu par des anticorps spécifiques. Par exemple, la sonde est modifiée par le N-acétoxy-N-2-acétylaminofluorène, ci-après dénommé AAF. Une telle sonde, ci-après dénommée « sonde ADN-AAF », qui comporte au moins un groupe N-2-acétylamino-fluorène fixé de façon covalente à au moins l'une de ses bases a été décrite dans la demande de brevet français n° 81 24131 (WO 83/02286). Elle est reconnaissable par des anticorps préalablement formés contre la N-2-(guanosine-8-yl)-acétylaminofluorène ou contre la sonde elle-même portant des résidus d'acétylaminofluorène. De tels anticorps sont ci-après appelés « anticorps anti-AAF ».

Le procédé décrit dans ce brevet, de détection de la séquence ou d'un fragment déterminé recherché d'acide nucléique éventuellement contenu dans une composition donnée, consiste à mettre en contact la susdite composition avec une sonde ADN-AAF contenant la séquence complémentaire appropriée dans des conditions telles qu'il y ait hybridation entre la sonde et la séquence d'acide nucléique ou de gène recherché, puis à révéler la séquence d'acide nucléique ou de gène.

On a également fixé le dérivé iodé de l'AAF, c'est-à-dire le 7-iodo-N-acétoxy-N-2-acétylaminofluorène, ci-après désigné AAIF, sur l'ADN (Fuchs et coll., Biochemistry, vol. 15, 1976, p. 3347 à 3351), afin d'étudier le modèle moléculaire obtenu à l'issue de la fixation. Mais la transposition de cette technique à la fabrication d'une sonde ADN-modifiée et susceptible d'être reconnue par des anticorps appropriés ne pouvait cependant pas être envisagée jusqu'à ce jour. En effet, en raison de l'insolubilité du dérivé iodé du N-2-(guanosine-8-yl)-acétylaminofluorène — du moins dans les conditions dans lesquelles, à la connaissance des inventeurs, celui-ci a pu être obtenu —, il n'était pas concevable de produire des anticorps contre cette molécule par immunisation d'un animal d'expérience tel que le lapin. De plus, les dimensions importantes au niveau moléculaire de l'atome d'iode pouvaient être considérées comme s'opposant a priori à l'utilisation de groupes de ce type pour le marquage d'une sonde, en raison de la réduction de la capacité d'hybridation que l'on pouvait en attendre.

Ces différentes constatations n'étaient donc pas de nature à inciter l'homme de l'art à se tourner vers l'AAIF en tant que marqueur pour la production de sondes de détection du genre sus-indiqué. Même si l'on fait abstraction du préjugé que l'on pouvait avoir à l'égard d'un tel type de marqueur, il restait à découvrir les moyens qui pouvaient permettre sa reconnaissance aisée. Or la présente invention repose sur la découverte que non seulement des anticorps préalablement formés contre l'AAF ou un ADN-AAF sont susceptibles de reconnaître le même ADN modifié par l'AAIF, mais encore que la sensibilité de détection de cet ADN-AAIF par les anticorps autres qu'AAF était beaucoup plus élevée, notamment de l'ordre de 10 fois, que la sensibilité de détection de l'ADN-AAF lui-même par ces mêmes anticorps. Il est en outre remarquable que la fixation de « grosses molécules » sur un ADN ne modifie guère la capacité de ces mêmes ADN, après dénaturation préalable, de s'hybrider avec une séquence complémentaire d'un ADN distinct. La sensibilité de détection d'ADN marqués par des anticorps anti-AAF d'un ADN marqué par AAIF, plus grande que lorsque le marqueur est constitué par le groupe AAF directement homologue de l'anticorps prend encore un relief particulièrement important si l'on tient compte de concentrations extrêmement faibles, voire d'extrême dilution, dans lesquelles on se trouve contraint d'opérer, dès lors que la séquence spécifique d'acide nucléique à détecter dans un échantillon biologique donné n'est souvent présente dans l'échantillon biologique à étudier qu'en quantités infinitésimales et que la détection elle-même s'effectue en général dans des conditions de dilution extrême. Par exemple, lorsque la séquence d'ADN spécifique recherchée contient de l'ordre de 3 000 paires de bases et que cette séquence appartient à un gène total d'une cellule haploïde ayant de l'ordre de $3 \times 10^{-9}$ paires de bases,

la concentration relative de la séquence spécifique recherchée dans l'ADN contenu dans l'échantillon biologique à étudier est de l'ordre de $10^{-6}$. A cette concentration extrêmement faible s'ajoutent souvent, notamment dans des essais de fractionnement préalable de l'ADN total étudié, les effets extrêmes de la dilution de la séquence recherchée, notamment lorsque ce fractionnement comporte des opérations du type électrophorèse ou analogue visant à séparer les différentes espèces moléculaires de l'ADN mis en œuvre par migrations différenciées selon leurs poids moléculaires respectifs, par exemple dans un gel d'agarose de polyacrylamide. Si l'on tient compte des quantités nécessairement limitées d'échantillon biologique qui peuvent ainsi être fractionnées, la multiplication de la sensibilité de détection par 10 peut devenir tout à fait essentielle. En effet, alors que des sondes marquées à l'AAF permettent déjà la détection de quantités de séquences spécifiques de l'ordre de 10 picogrammes, la même sonde marquée à l'AIF sera apte à déceler des quantités de l'ordre de 1 picogramme.

L'ensemble de ces propriétés favorables fait donc de l'AAIF un remarquable marqueur pour des sondes d'acide nucléique telles qu'elles ont été envisagées plus haut.

L'invention concerne toute sonde ainsi formée, plus particulièrement toute sonde clonée comportant une séquence déterminée d'acide nucléique complémentaire de la séquence spécifique recherchée insérée dans tout ou partie de l'acide nucléique d'un vecteur, notamment plasmide ou phage ayant permis la réalisation du clonage selon des techniques classiques en matière de génie génétique.

Notamment l'acide nucléique du vecteur qui avait été utilisé pour le clonage était hétérologue vis-à-vis de la susdite séquence déterminée d'acide nucléique, c'est-à-dire qu'il est d'une origine différente de celle de ladite séquence, c'est-à-dire qu'il provient d'un micro-organisme différent.

L'invention concerne encore d'une façon générale des nécessaires ou kits destinés à la recherche systématique de séquences déterminées de nucléotides dans un échantillon biologique déterminé, voire même plus généralement dans toutes préparations formées in vitro, par exemple un cADN résultant de la transcription reverse d'un ARN en présence des désoxyribonucléotides appropriés et d'une reverse-transcriptase.

Le nécessaire selon l'invention, permettant la détection et la caractérisation d'une séquence ou d'un fragment d'acide nucléique déterminé dans une composition susceptible de la contenir, comprend :

— une sonde contenant une séquence complémentaire d'acide nucléique recherché, ladite sonde comportant au moins un groupe AAIF fixé de façon covalente à au moins l'une de ses bases, et

— des anticorps anti-AAF.

Bien entendu le nécessaire selon l'invention peut comprendre tous les autres éléments nécessaires à son utilisation, notamment les réactifs pour préparer les solutions tampons, les réactifs appropriés pour extraire s'il y a lieu les ADN à étudier à partir de milieux cellulaires et, lorsque les susdits anticorps anti-AAF ne sont pas eux-mêmes modifiés pour les rendre directement visualisables, des anticorps ou autres polypeptides distincts, eux-mêmes capables de réagir avec les anticorps anti-AAF et eux-mêmes porteurs d'un marqueur aisément visualisable. La visualisation peut être réalisée par tout moyen approprié. De préférence, les seconds anticorps susdits ou polypeptides correspondants sont à leur tour modifiés par une molécule fluorescente ou par une enzyme, de préférence choisie parmi celles auxquelles correspond un substrat qui, lorsqu'il est modifié par l'enzyme, donne lieu à des modifications repérables par des moyens colorimétriques ou spectrophotométriques de l'absorption par ces substrats d'une ou plusieurs radiations lumineuses déterminées.

Le procédé selon l'invention est caractérisé par le fait que l'on met en contact avec la composition présumée contenir une séquence ou un fragment déterminé d'acide nucléique, dans des conditions permettant l'hybridation éventuelle, une sonde contenant un acide nucléique complémentaire susceptible de s'hybrider avec la séquence d'acide nucléique ou le gène recherché, la sonde portant au moins un groupe AAIF fixé de façon covalente à au moins l'une de ses bases et que, après séparation éventuelle de la sonde non hybridée, on révèle la sonde hybridée par mise en contact avec des anticorps anti-AAF.

Les sondes selon l'invention peuvent contenir des fragments spécifiques d'ADN ou d'ARN, cellulaires ou viral, ou encore un fragment déterminé de cADN.

Elles peuvent encore, notamment dans le cadre du procédé et du nécessaire selon l'invention être constituées par des fragments d'ADN plus importants, notamment des fragments du génome, voire même les génomes entiers de micro-organismes, notamment bactériens ou viraux, marqués par l'AAIF. Ces sondes seront alors utilisables dans le dépistage ou la détection de ces micro-organismes dans tout milieu susceptible de les contenir.

L'ADN-AAIF utilisé comme sonde selon l'invention est mis en présence de l'ADN à étudier dans des conditions permettant le réappariement de séquences complémentaires, ce qui peut naturellement impliquer une dénaturation préalable dans des conditions bien connues des ADN susceptibles de s'hybrider mutuellement.

Avantageusement, des quantités déterminées du produit de réaction sont ensuite déposées et fixées dans des conditions bien connues du spécialiste, sur un filtre de cellulose ou support analogue.

En variante, des quantités déterminées de la composition contenant l'ADN à étudier sont déposées et fixées sur un tel support préalablement à la réalisation de l'hybridation. Celle-ci est alors réalisée directement sur le support. Après hybridation, l'ADN-AAIF non hybridé de façon spécifique est éliminé par rinçage avant que l'on ne procède à la détection des hybrides formés, notamment par leur mise en présence avec des anticorps anti-AAF, lesquels peuvent alors se fixer sur la sonde à la fois modifiée et hybridée avec la séquence d'ADN recherchée, lorsque celle-ci est présente dans la composition utilisée.

Après rinçage des anticorps excédentaires encore présents, les anticorps fixés peuvent être soit précipités, soit révélés.

De préférence, la révélation est faite au moyen d'anticorps distincts susceptibles de réagir avec les anticorps anti-AAF, ces anticorps distincts étant alors marqués par une enzyme dont on peut ensuite détecter ou doser l'activité vis-à-vis d'un substrat spécifique. Avantageusement, on utilisera celles des enzymes qui sont susceptibles d'induire une réaction colorée au niveau des substrats correspondants.

La révélation à l'aide d'enzymes donnant des réactions colorées est très rapide.

La méthode est très sensible surtout si on utilise des systèmes amplificateurs (chapelets, arbres ou boules d'anticorps associés à des enzymes) de sorte qu'elle permette de localiser des gènes après hybridation in situ sur des chromosomes, par exemple dans le cas de diagnostics prénatals.

La méthode peut être quantitative par mesure de l'intensité de la coloration.

On peut également, en lieu et place des susdits anticorps distincts, utiliser par exemple des immunoglobulines marquées ou de la protéine A de Staphylococcus aureus qui peuvent être utilisées dans des conditions analogues, d'ailleurs bien connues du technicien.

Des caractéristiques supplémentaires de l'invention apparaitront encore au cours de la description qui suit d'un exemple type de mise en œuvre procédé.

On fait usage des matières et des méthodes suivantes.

Les ADN de phage λ proviennent de Biolabs Inc. (Nelle Angleterre).

L'acide ribonucléique ribosomique provient de foie de bœuf et les réactifs pour la démonstration à la phosphatase alcaline (sel RR Fast Blue, Naphtol AS-MX phosphate) proviennent de la firme Sigma.

Les filtres de nitrocellulose, de type BA-85, proviennent de Schleicher et Schull. Les cellules Staph. A fixées par la formaline (commercialisées sous le nom Immunoprécipitine) proviennent de Bethesda Research Laboratories.

Les kits de « nick-translation » (réf. N-5 000) et les nucléotides marqués radio-activement $\alpha$-$^{32}$ p-dCTP, 800 Ci/mmole) proviennent d'Amersham.

L'AAF et l'AAIF sont synthétisés selon les articles parus dans Biochemistry, 15, 3 347-3 351 (FUCHS et coll.) et Biochemistry, 17, 2 561-2 567 (LEFEVRE et coll.). Ils sont conservés dans des tubes enveloppés dans des feuilles d'aluminium, sous azote à — 20 °C.

Les anticorps anti-ADN-AAF et anti-Guo-AAF sont obtenus comme décrit dans Febs Lett., 92, 207-210 (LENG et coll.), Biochemistry, 18, 1 328-1 332 (SAGE et coll.) et dans Nucleic Acids Res., 6, 733-744 (GUIGUES et coll.).

Les anticorps liés à la peroxydase et à la phosphatase alcaline proviennent de Miles Laboratories et de l'Institut Pasteur.

Le plasmide et les clones M13 avec les insertions d'ADN cellulaire de dzeta globine humaine proviennent de l'unité INSERM U91, Hôpital Henir Mondor, Créteil, France et du Département de Médecine de l'Université de Médecine de Yale (New Haven — Etats-Unis). Le 4p7-7 porte un fragment d'ADN cellulaire de 464 paires de bases de dzeta globine inséré sur le pBR 322 au site Pst I. Le même fragment est inséré dans le M13 (DNA 1, 355-363, COHEN-SOLAL et coll.).

L'ADN M13 monocaténaire est préparé selon Messing et coll. dans Nucleic Acids Res., 9, 309-321.

Le clone pWE6 provient du Centre de Recherches de Biochimie et de Génétique Cellulaire du CNRS, Toulouse, France. Il contient un ADN ribosomique 45 S de souris de 6,6 kilobases inséré dans pBR 322 au site Eco RI (Nucleic Acids Res., 10, 5 273-5 283, MICHOT et coll. et Nucleic Acids Res., 11, 3 375-3 391, MICHOT et coll.).

Préparation de la sonde

1° Utilisation d'acides nucléiques bicaténaires

Les acides nucléiques à modifier sont réduits en fragments d'environ 1 000 paires de bases (pb) par sonication et dissous dans du tampon citrate de sodium (2 mM, pH 7) à une concentration d'environ 500 µg/ml. Après dénaturation par chauffage (100 °C, 5 minutes) et refroidissement rapide dans la glace, on ajoute à cette solution 1/10 de volume d'une solution d'AAIF dans de l'éthanol. La quantité totale d'AAIF ajouté doit être égale à environ 3 fois le poids d'acides nucléiques à modifier. Le mélange est incubé 3 heures à 37 °C à l'abri de la lumière. Après incubation, l'excès d'AAIF est éliminé par trois extractions à l'éther éthylique froid et la préparation est traitée avec du tampon borate (50 mM, pH 9) à 100 °C pendant 3 minutes, ceci pour éliminer les liaisons inter-chaînes qui auraient pu se produire. Après neutralisation avec du tampon Tris HCl (100 mM, pH 7) la préparation est prête à être utilisée pour l'hybridation. Elle peut être conservée à + 4 °C, ou de préférence à — 20 °C, pendant plusieurs années.

Lorsqu'on utilise de l'ADN radio-actif, l'ADN « nick-translaté » est précipité par de l'éthanol redissous dans du tampon de citrate et dénaturé par la chaleur. On traite des aliquots comme indiqués ci-dessus en omettant l'étape de sonication. L'ADN témoin est traité avec de l'éthanol pur. Après traitement, on ajoute de l'EDTA jusqu'à une molarité finale de 2 mM et les acides nucléiques modifiés sont conservés à l'abri de la lumière à 4 °C.

2° Utilisation d'acides nucléiques monocaténaires

On opère comme indiqué au 1°, sans effectuer l'étape de sonication.

Détermination du pourcentage des bases modifiées

Le pourcentage des bases modifiées est déterminé en mesurant l'absorption à 310 nm et 260 nm comme décrit dans Biochemistry, 11, 2 659-2 666, FUCHS et coll. et Febs Lett., 34, 295-298, FUCHS et coll. Lorsque cette méthode photométrique ne peut pas être utilisée en raison d'échantillons trop petits, on dépose sur un filtre de nitrocellulose plusieurs dilutions d'échantillons à tester et d'un échantillon déjà mesuré. Après coloration immunochimique, on compare les intensités de couleur des taches.

Hybridation

Les essais ont été faits sur de l'ADN adsorbé à des filtres en nitrocellulose par des techniques courantes (filtration à l'aide d'un appareil du type « Hybridot » commercialisé par Bethesda Research Laboratories ou transfert par capillarité selon la méthode de Southern, décrite dans J. Mol. Biol., 98, p. 503, 1975, mais l'utilisation d'autres supports peut être envisagée (par exemple filtres en nylon du type « Biodyne » commercialisés par PALL, papier DBM (diazobenzyloxymethyl) (Cell, vol. 5, p. 301, Noyes et Stark, 1975 et Alwine et coll. 1977, PNAS, 1974, p. 5350, Kemp coll.).

Les filtres sont préhybridés pendant 2 heures à 65 °C dans une solution contenant :

| | |
|---|---|
| — NaCl | 300 mM |
| — citrate de sodium, pH 7 | 30 mM |
| — réactif commercialisé sous la désignation Ficoll 400 par la Société Pharmacia Fine Chemicals | 0,1 % |
| — polyvinylpyrrolidone 350 | 0,1 % |
| — glycine | 0,1 % |

L'hybridation avec la sonde modifiée (ADN-AAIF ou ARN-AAIF) est faite à 65 °C pendant le temps voulu dans la solution suivante :

| | |
|---|---|
| — NaCl | 300 mM |
| — citrate de sodium, pH 7 | 30 mM |
| — Ficoll 400 | 0,02 % |
| — Polyvinylpyrrolidone 350 | 0,02 % |
| — glycine | 0,02 % |
| — KH$_2$PO$_4$, pH 7 | 25 mM |
| — EDTA, PH 7 | 2 mM |
| — dodécylsulfate de sodium (SDS) | 0,5 % |

La durée de l'hybridation dépend de la concentration de la sonde et de la complexité de la séquence à rechercher. Elle peut être calculée par des méthodes connues. L'utilisation de produits accélérant la renaturation des acides nucléiques (par exemple le sulfate de dextrane) ou abaissant la température d'hybridation (par exemple la formamide) est possible.

Après hybridation, les filtres sont rincés pour éliminer l'excès de sonde en suivant des protocoles couramment utilisés et décrits dans la littérature.

Détection des sondes

Les filtres hybridés sont soumis aux traitements suivants :
— saturation en protéines par incubation pendant une heure, à température ambiante dans une solution contenant :

| | |
|---|---|
| • sérum de veau | 20 % |
| • NaCl | 300 nM |
| • citrate de Na, pH 7 | 30 nM |
| • NP 40 | 1 % |

(solution saturante) ;
— mise en présence du premier anticorps (anticorps reconnaissant les modifications AAIF) pendant une heure à température ambiante. Cet anticorps est dilué avec la solution saturante. Le rapport de dilution dépend de l'anticorps utilisé ;
— rinçage dans une solution contenant :

| | |
|---|---|
| • NaCl | 300 mM |
| • citrate de sodium, pH 7 | 30 mM |
| • NP 40 | 1 % |

(solution de rinçage) ;
— incubation pendant une heure à température ambiante avec le deuxième anticorps, reconnaissant le premier anticorps (si le premier anticorps est un anticorps de lapin, le second anticorps peut être, par exemple, un anticorps anti IgG de lapin lié à de la phosphatase alcaline).

Le second anticorps est dilué dans la solution saturante, dans un rapport qui dépend de l'anticorps utilisé.

— Rinçage en utilisant la même solution de rinçage que celle indiquée ci-dessus ;

— révélation du second anticorps par des techniques connues (par exemple réaction enzymatique colorée de la phosphatase alcaline).

Sensibilité de la méthode

Les essais ont été faits en utilisant comme cible de l'ADN viral (phage λ) ou des gènes humains (dzeta globine fœtale, 460 pb) ou de souris (ADN ribosomique, 6,6 kb) clonés dans des plasmides bactériens (pBR 322), et comme sonde de l'ADN bicaténaire (ADN de phage λ ou de plasmide pBR 322), monocaténaire (ADN de phage M13 portant une insertion de 464 pb de dzeta globine humaine), ou de l'ARN (ARN ribosomique de bœuf).

Pour la détection des sondes, le premier anticorps était un anticorps de lapin anti-guanosine-AAF (sérum brut) à une dilution de 1/200 et le second anticorps, un anticorps anti-IgG de lapin lié à de la phosphatase alcaline et utilisé à une dilution de 1/400.

Dans ces conditions, les sensibilités obtenues ont été les suivantes :

— pour des sondes d'ADN bicaténaire utilisé à une concentration supérieure ou égale à 500 ng/ml $(500 \times 10^{-9}$ g/ml) : sensibilité meilleure que 2 pg d'ADN cible $(2 \times 10^{-12}$ g) ;

— pour des sondes d'ADN monocaténaire utilisé à une concentration supérieure ou égale à 100 ng/ml : sensibilité meilleure que 2 pg d'ADN cible ;

— pour des sondes d'ARN ribosomique à une concentration de 200 ng/ml : sensibilité voisine de 200 pg.

On rappelle qu'une sensibilité de 2 pg d'ADN cible correspond à la détection d'un gène de 1 000 pb dans 7 μg d'ADN humain total. Cette sensibilité est du même ordre de grandeur que celle exigée pour le diagnostic prénatal de l'anémie falciforme.

Comparaison des sensibilités respectives des sondes ADN-AAF et ADN-AAIF

Les acides ribo- ou déoxyribonucléiques réagissent facilement in vitro à pH neutre avec l'AAF et l'AAIF. Dans les conditions décrites plus haut, les dérivés sont liés principalement au carbone 8 du résidu de la guanine par une liaison covalente. Les anticorps obtenus en immunisant des lapins avec de l'ADN-AAF et Guo-AAF reconnaissent spécifiquement l'ADN modifié par l'AAF. Ils reconnaissent également l'ADN et l'ARN modifiés par l'AAIF. Les résultats du Tableau I ci-après montre une quantité plus importante d'ADN trouvée dans le précipité lorsque l'ADN est modifié par l'AAIF.

De petites quantités d'ADN normal sont précipitées par des anticorps anti-ADN-AAF. Pour différencier l'ADN modifié du non modifié, on utilise de préférence les anticorps anti-Guo-AAF car les résultats obtenus sont meilleurs. En effet, seules des quantités négligeables d'ADN non modifié sont précipitées par des anticorps anti-Guo-AAF purifiés. Ceci permet d'utiliser les sondes pour la séparation des séquences de gènes spécifiques à partir de mélanges complexes.

La détection des acides nucléiques modifiés par des techniques nucléochimiques est faite de la façon suivante. L'ADN modifié est lié aux filtres de nitro-cellulose et les filtres sont ensuite traités comme indiqué plus haut. Etant donné que le nombre de paramètres à étudier est très important, on choisit dans les expériences de petits filtres ronds, de 25 mm de diamètre, car ils sont faciles à manipuler. En utilisant de l'ADN modifié par l'AAIF (5 % de base modifiée), la limite de sensibilité est inférieure à 1 pg lorsque les deuxièmes anticorps sont liés à la phosphatase alcaline et environ 8 pg lorsqu'ils sont liés à la peroxydase.

En ce qui concerne la température de fusion de l'ADN, elle est approximativement diminuée de 1,1 °C et 0,4 °C, respectivement pour 1 % de base modifiée par de l'AAF et de l'AAIF (Biochemistry, 15, 3 347-3 351, FUCHS et coll. : Febs Lett.. 34, 295-298, FUCHS et coll. ; Biochemistry, 6, 177-182, KRIEK et coll. ; Biochem. Biophys. Acta, 232, 436-450, KAPULER et coll.).

Des hybridations par taches ont été faites pour évaluer l'effet des modifications de l'AAF et de l'AAIF sur des sondes bicaténaires d'ADN. Des filtres de nitrocellulose avec trois taches d'ADN de pBR 322 (422 pg, 2 ng, 20 ng) et une tache d'ADN (1 ng) sont hybridés avec des sondes d'ADN radio-actives, soit non modifiés, soit avec environ 5 % de base modifiée par l'AAF ou l'AAIF. Après hybridation, des ensembles donnés de filtres sont lavés avec plus ou moins de force, séchés et auto-radiographiés pendant 18 heures. Aucune hybridation non spécifique n'a pu être observée, même avec le lavage de plus faible force (NaCl/citrate de sodium, 15 minutes à la température ambiante).

La stabilité de l'hybride est testée sur les filtres avec des taches d'ADN (10 pg, 100 pg, 1 ng, 10 ng) qui sont lavées à des forces différentes après hybridation :

— soit avec les sondes d'ADN modifiées radioactives ;

— soit avec les sondes de contrôle.

A chaque degré de force de lavage, on sèche un ensemble de filtres et on les auto-radiographie. On n'a pu ici remarquer aucune différence appréciable entre les trois sondes. On ne remarque aucune tache sur les filtres hybridés avec une sonde d'ADN témoin quelle que soit la force de lavage. La tache correspondante à 10 ng est légèrement visible sur le filtre hybridé avec une sonde modifiée à l'AAF. Avec une sonde modifiée à l'AAIF, la tache correspondante à 1 ng est également visible.

Pour chacune des expériences indiquées ci-dessus, chacune des concentrations de sonde est de 2 ng/ml, l'activité spécifique est de $5 \times 10^7$ cpm/µg et le temps d'auto-radiographie est de 18 heures. Dans ces conditions, la sensibilité de la détection obtenue avec les sondes modifiées par l'AAF et l'AAIF sont environ de 1/100 et 1/10 comparée à celle obtenue par auto-radiographie.

Etant donné que n'importe quel type d'acide nucléique peut être modifié soit par l'AAF ou l'AAIF, on a testé la possibilité d'utiliser des sondes d'ADN M13 monocaténaire et des sondes d'ARN immunonucléique dans des expériences d'hybridation. Pour l'hybridation avec l'ADN M13 monocaténaire, chaque filtre contient une tache de témoin ADN de phage λ et des quantités décroissantes d'ADN du plasmide 4p7-7. L'ADN monocaténaire d'un phage de recombinant de M13 avec la même insertion de 464 paires de bases de dzeta globine est modifié par l'AAF ou l'AAIF (5 % de paires de bases modifiées). Plusieurs concentrations de sonde de 125 à 1 500 ng/ml sont testées. L'hybridation et le lavage sont effectués à 65 °C. Dans ce cas, la concentration des séquences hybridables de la sonde est d'environ 33 ng/ml et les séquences cibles sur les taches sont comprises entre 660 et 5 ng. Toutes les taches sont colorées sauf le témoin négatif. La coloration des taches est plus intense avec la sonde modifiée par l'AAIF.

Des essais semblables sont faits avec des sondes d'ARN modifiées par l'AAIF.

De l'ARN ribosomiques de foie de bœuf (Sigma, référence R 5 502) est dissous dans du tampon de citrate (2 mM, pH 6, 7), soniqué brièvement pour réduire sa taille à des fragments d'environ 1 000 bases et modifié par l'AAIF. On fait des taches avec de l'ADN de pBR 322 (100 ng) pour avoir le témoin négatif, et des quantités décroissantes d'ADN de pWE 6, un plasmide recombinant avec une insertion d'ADN ribosomique 45 S de souris de 6,6 kb. La quantité de séquences hybridables sur les taches est comprise entre environ 30 ng et 230 pg. On utilise des concentrations de sonde différentes allant de 200 à 2 300 ng/ml. On n'observe que de légères différences dans les intensités de coloration avec les concentrations plus élevées de sonde. Lorsqu'on utilise des sondes immunonucléiques à hautes concentrations, le bruit de fond des filtres reste très bas. La possibilité d'utiliser des concentrations élevées de sonde est intéressante lorsqu'on souhaite faire des hybridations courtes.

De l'ensemble de ces résultats, il apparaît que les propriétés des acides nucléiques modifiés par l'AAIF les rendent appropriés pour la détection de séquences spécifiques.

L'utilisation du dérivé iodé AAIF à la place de l'AAF apporte un gain de sensibilité de 10 fois.

Par ailleurs, lorsque les deuxièmes anticorps utilisés sont liés à la phosphatase alcaline, la sensibilité est du domaine du picogramme.

L'invention ne se limite évidemment pas aux modes de réalisation décrits ci-dessus à titre d'exemples et l'homme de l'art peut y apporter des modifications sans pour autant sortir du cadre des revendications ci-après.

Au titre des variantes utilisables au niveau de la détection des hybrides formés avec la sonde selon l'invention, on citera :

— la révélation (par la radio-activité) des hybrides formés, par exemple grâce à l'utilisation d'anticorps anti-ADN-AAF rendus radio-actifs par de l'iode 125 ou 131 ou de protéine A radio-active, qui va se fixer sur les anticorps.

Enfin, au titre des variantes d'applications possibles, on citera l'application de la sonde selon l'invention à la purification d'ADN complémentaire contenu dans une composition initiale, notamment au moyen :

— de protéine A associée à un support solide (par exemple constitué de billes d'agarose) ;

— d'anticorps précipitants associés ou non à un support solide (billes d'agarose, de latex, etc.) ; pour assurer la précipitation sélective de l'hybride formé.

# 0 158 758

Tableau I

Pourcentages d'ADN immunoprécipité

| Anticorps | ADN (témoin) | ADN–AAF (∿5 % bases modifiées) | ADN–AAIF (∿5 % bases modifiées) |
|---|---|---|---|
| Lapin (sérum normal) | < 0,1 | < 0,1 | < 0,1 |
| Anti-Guo-AAF (sérum entier) | 0,2 | 44,7 | 81,9 |
| Anti-Guo-AAF (purifié) | 0,1 | 41,5 | 71,9 |
| Anti-ADN-AAF (purifié) | 1,0 | 66,8 | 93,6 |

**Revendications**

1. Nécessaire pour la détection de séquences déterminées de nucléotides ou plus généralement d'ADN déterminés dans un échantillon biologique susceptible de le contenir, caractérisé en ce qu'il comprend :
— une sonde contenant une séquence complémentaire de l'acide nucléique recherché, ladite sonde comportant au moins un groupe 7-iodo-N-2-acétylaminofluorène (groupe AAIF) fixé de façon covalente à l'une au moins de ses bases et
— des anticorps susceptibles de réagir avec soit la N-2-(guanosine-8-yl)-acétylaminofluorène, soit avec des anticorps préalablement préparés contre une séquence d'acide nucléique modifiée par un groupe N-2-acétylaminofluorène (anticorps AAF).

2. Nécessaire selon la revendication 1, caractérisé en ce qu'il comprend en outre des moyens de visualisation des anticorps anti-AAF.

3. Nécessaire selon la revendication 2, caractérisé en ce que ces moyens de visualisation sont constitués par des anticorps distincts ou autres polypeptides distincts, eux-mêmes. capables de réagir avec les anticorps anti-AAF et eux-mêmes porteurs d'un marqueur, tel qu'une enzyme ou une molécule fluorescente.

4. Procédé de détection et de caractérisation d'une séquence ou d'un fragment d'acide nucléique déterminé dans une composition susceptible de les contenir, caractérisé par la mise en contact des acides nucléiques de ladite composition, dans des conditions permettant l'hybridation éventuelle, avec une sonde telle que définie dans l'une quelconque des revendications 1 à 3, contenant une séquence ou un acide nucléique complémentaire, puis la visualisation de l'hybride éventuellement formé entre la sonde et un acide nucléique initialement contenus dans ladite composition.

5. Sonde clonée comportant une séquence déterminée d'acide nucléique complémentaire d'une séquence susceptible d'être recherchée parmi les acides nucléiques contenus dans une composition donnée caractérisée par le fait que cette séquence déterminée est insérée dans un vecteur ou fragment de vecteur hétérologue vis-à-vis de ladite séquence et en ce que cette sonde est marquée par des groupes AAIF.

**Claims**

1. Kit for the detection of predetermined nucleotide sequences or more generally particular DNAs in a biological sample liable to contain it, characterized in that it comprises :
— a probe containing a sequence complementary to the nucleic acid which is sought, said probe comprising at least one 7-iodo-N-acetylaminofluorene group (AAIF group) covalently fixed at one at least of its bases and
— antibodies which can react with either N-2-(guanosine-8-yl)-acetylaminofluorene or with antibodies previously prepared against a nucleotidic acid sequence modified by a N-2-acetylaminofluorene group (antibodies AAF).

2. Kit according to claim 1, characterized in that it comprises in addition means for visualizing anti-AAF antibodies.

3. Kit according to claim 2, wherein these visualization means are constituted by distinct antibodies or other distinct polypeptides, themselves capable of reacting with anti-AAF antibodies and themselves bearing a marker, such as an enzyme or a fluorescent molecule.

4. Method for detecting and characterizing a sequence of a particular nucleic acid fragment in a composition which can contain them, characterized by contacting the nucleic acids of said composition, under conditions enabling possible hybridization, with a probe such as defined in any one of claims 1 to 3, containing a sequence or a complementary nucleic acid, then visualizing the hybrid possibly formed between the probe and a nucleic acid initially contained in said composition.

5. Cloned probe comprising a predetermined sequence of nucleic acid complementary with a sequence which can be sought among the nucleic acids contained in a given composition characterized by the fact that this predetermined sequence is inserted in a vector or vector fragment heterologous with respect to said sequence and that this probe is marked by AAIF groups.

## Patentansprüche

1. System zum Nachweis von bestimmten Nukleotidsequenzen oder ganz allgemein, von bestimmten DNAs in einer biologischen Probe, die diese enthalten kann, dadurch gekennzeichnet, daß es

— eine Sonde, welche eine komplementäre Sequenz zu der herauszusuchenden Nucleinsäure enthält, wobei diese Sonde mindestens eine 7-Jod-N-2-acetylaminofluorengruppe (AAIF-Gruppe) aufweist, die an mindestens eine ihrer Basen kovalent gebunden ist und

— Antikörper, die entweder mit (Guanosin-8-yl)-N-2-acetylaminofluoren oder mit zuvor hergestellten Antikörpern gegen eine mit einer N-2-Acetylaminofluorengruppe modifizierte Nucleinsäuresequenz (AAF-Antikörper), reagieren können, umfaßt.

2. System nach Anspruch 1, dadurch gekennzeichnet, daß es weiterhin Mittel zum Sichtbarmachen der Anti-AAF-Antikörper aufweist.

3. System nach Anspruch 2, dadurch gekennzeichnet, daß diese Mittel zum Sichtbarmachen aus bestimmten Antikörpern oder anderen bestimmten Polypeptiden bestehen, die in der Lage sind, mit den Anti-AAF-Antikörpern zu reagieren und einen Marker in Form eines Enzyms oder fluoreszierenden Moleküls aufweisen.

4. Verfahren zum Nachweis und zur Charakterisierung einer Sequenz oder eines bestimmten Nucleinsäurefragments in einer Zusammensetzung, die diese enthalten kann, dadurch gekennzeichnet, daß man die Nucleinsäuren dieser Zusammensetzung mit einer Sonde nach einem der Ansprüche 1 bis 3, welche eine komplementäre Sequenz oder Nucleinsäure enthält, unter Bedingungen, die eine mögliche Hybridisierung erlauben, in Kontakt bringt und dann den gegebenenfalls zwischen der Sonde und der anfangs in der Zusammensetzung enthaltenden Nucleinsäure gebildeten Hybrid sichtbar macht.

5. Clonierte Sonde, enthaltend eine bestimmte Nucleinsäuresequenz, die zu einer Sequenz, welche unter den in einer gegebenen Zusammensetzung enthaltenden Nucleinsäuren herausgesucht werden kann, komplementär ist, dadurch gekennzeichnet, daß die bestimmte Sequenz in einem Vektor oder Vektorfragment, der zu dieser Sequenz heterolog ist, eingesetzt ist, und daß die Sonde durch AAIF-Gruppen markiert ist.